# FASCICULE DE BREVET EUROPEEN

(11) **EP 3 561 080 B1**
(45) Date de publication et mention de la délivrance du brevet: **02.08.2023**
(21) Numéro de dépôt: 18305502.9
(22) Date de dépôt: 23.04.2018
(51) Int. Cl.: C13K 11/00, B01D 15/18, C12N 9/92, B01D 15/36

(54) **PROCEDE DE FABRICATION DE FRUCTOSE A PARTIR DE GLUCOSE**
HERSTELLUNGSVERFAHREN VON FRUKTOSE AUS GLUKOSE
METHOD FOR PRODUCING FRUCTOSE FROM GLUCOSE

(43) Date de publication de la demande: 30.10.2019
(73) Titulaire: Novasep Process Solutions, 01700 Saint-Maurice-de-Beynost (FR)
(72) Inventeur: BRICHANT, Damien, 56370 Sarzeau (FR); VALERY, Eric, 69003 Lyon (FR)
(74) Mandataire: Bandpay & Greuter

(56) Documents cités:
- WO-A1-2016/061037
- CN-A- 104 630 312
- CN-A- 105 177 087
- FR-A1- 2 668 775
- FR-A1- 2 912 036
- US-A- 5 234 503

## Description

### DOMAINE DE L'INVENTION

La présente invention concerne un procédé de production d'une composition de fructose à partir d'une composition initiale comprenant du glucose, ainsi qu'une installation adaptée à la mise en oeuvre de ce procédé.

### ARRIERE-PLAN TECHNIQUE

Dans l'industrie agroalimentaire, il est fait un usage important de compositions à base de fructose, notamment connues sous le sigle HFS pour « *High Fructose Syrup* ». En particulier, on connaît sous la désignation HFS 55 une composition comprenant environ 55 % en masse de fructose par rapport à la matière sèche totale, et sous la désignation HFS 95 une composition comprenant au moins 95 % en masse de fructose par rapport à la matière sèche totale.

Il est connu de fabriquer les compositions HFS 55 et HFS 95 par isomérisation à partir d'une composition à base de glucose. Traditionnellement, le procédé de fabrication inclut une première évaporation pour concentrer la composition à base de glucose, puis une étape d'isomérisation, puis une autre évaporation pour effectuer une deuxième concentration, puis une purification chromatographique permettant de séparer un flux enrichi en fructose d'un flux enrichi en glucose, et une autre évaporation pour effectuer une troisième concentration, à partir du flux enrichi en fructose. Un exemple de ce type de procédé est décrit par US 5 234 503.

Ce procédé nécessite une installation relativement lourde et implique une consommation énergétique importante.

Il existe donc un besoin de produire des compositions à base de fructose, telles que la composition HFS 55 ou la composition HFS 95, avec une plus grande efficacité (et par exemple avec une moindre consommation énergétique) et/ou avec des dimensions de colonnes de chromatographie réduites.

### RESUME DE L'INVENTION

L'invention concerne un procédé de production d'une composition de fructose comprenant les étapes successives suivantes :
- fourniture d'une composition initiale comprenant du glucose ;
- concentration de la composition initiale par évaporation d'eau pour obtenir une composition initiale concentrée ;
- isomérisation du glucose en fructose à partir de la composition initiale concentrée, permettant d'obtenir une composition intermédiaire ;
- purification de la composition intermédiaire dans un système de chromatographie multicolonnes, permettant d'obtenir un raffinat riche en glucose et un extrait riche en fructose ;
- concentration de l'extrait par évaporation d'eau ;
la composition intermédiaire n'étant pas soumise à une étape de concentration par évaporation d'eau entre l'étape d'isomérisation et l'étape de purification. De plus, le raffinat est recyclé et ajouté à la composition initiale avant l'étape de concentration de la composition initiale.

Selon une variante de cette invention, la concentration massique en matière sèche de la composition intermédiaire à l'issue de l'étape d'isomérisation est égale à la concentration massique en matière sèche de la composition intermédiaire soumise à l'étape de purification à 5 % près.

Dans certains modes de réalisation, le procédé de l'invention comprend en outre une étape d'élimination de couleur résiduelle de l'extrait avant l'étape de concentration de l'extrait, de préférence par résine échangeuse d'ions et/ou par charbon actif ainsi que, de préférence, une étape de filtration stérile.

Dans certains modes de réalisation, le procédé de l'invention comprend en outre une étape de déminéralisation entre l'étape d'isomérisation et l'étape de chromatographie.

Dans certains modes de réalisation du procédé de l'invention, la purification est effectuée à une température supérieure ou égale à 50°C, de préférence de 55 à 65°C, et de préférence encore d'environ 60°C.

Dans le procédé de l'invention, le raffinat est recyclé et ajouté à la composition initiale avant l'étape de concentration de la composition initiale.

Dans certains modes de réalisation du procédé de l'invention :
- la composition initiale présente une concentration massique en matière sèche de 25 à 35 %, de préférence d'environ 31 % ; et/ou
- la composition initiale concentrée présente une concentration massique en matière sèche supérieure ou égale à 40 % ; et/ou
- la composition intermédiaire comprend au moins 40 % en masse de fructose par rapport à la matière sèche totale ; et/ou
- la composition de fructose produite présente une concentration massique en matière sèche supérieure ou égale à 75 %, de préférence de 77 % environ.

Dans certains modes de réalisation du procédé de l'invention, la composition intermédiaire qui est soumise à l'étape de purification présente une concentration massique en matière sèche de 45 à 55 %, et de préférence d'environ 50 %.

Dans certains modes de réalisation du procédé de l'invention :
- la composition de fructose produite contient une proportion massique de fructose, par rapport à la matière sèche totale, supérieure ou égale à 95 %, de préférence supérieure ou égale à 98 % ; ou
- une partie de la composition intermédiaire est prélevée avant l'étape de purification et ajoutée à l'extrait avant l'étape de concentration de l'extrait, la composition de fructose produite contenant de préférence une proportion massique de fructose, par rapport à la matière sèche totale, de 50 à 60 %, de préférence encore de 54 à 56 % ; et le rapport massique de la partie de composition intermédiaire prélevée sur la composition intermédiaire totale étant de préférence de 0,4 à 0,6, de préférence encore de 0,45 à 0,55, de préférence de 0,48 à 0,52.

Dans certains modes de réalisation du procédé de l'invention, la concentration massique en matière sèche de la composition intermédiaire varie de moins de 5%, et de préférence reste essentiellement constante, entre la fin de l'étape d'isomérisation et le début de l'étape de purification.

Dans certains modes de réalisation du procédé de l'invention, le système de chromatographie multicolonnes comprend de 4 à 6 cellules ; et/ou comprend des colonnes ayant une longueur de 1,0 à 2,6 m, de préférence de 1,4 à 2,0 m.

Dans certains modes de réalisation, le procédé de l'invention comprend l'injection d'eau en tant qu'éluant dans l'étape de purification, et :
- le rapport du débit massique d'éluant sur le débit massique de matière sèche de composition de fructose produite est de 0,5 à 1,3, de préférence de 0,6 à 1,2 ; et/ou
- le volume d'éluant injecté est de 0,12 à 0,24 BV.

Dans certains modes de réalisation du procédé de l'invention, le volume de composition intermédiaire soumis à l'étape de purification est de 0,13 à 0,40 BV.

Dans certains modes de réalisation du procédé de l'invention, la composition intermédiaire soumise à l'étape de purification contient une proportion massique de fructose supérieure à 42 %.

Dans certains modes de réalisation du procédé de l'invention, le système de chromatographie multicolonnes comprend une pluralité de colonnes et des liaisons fluidiques inter-colonnes, et la vitesse des fluides dans les liaisons fluidiques inter-colonnes est supérieure à 0,5 m/s, de préférence supérieure à 1 m/s et de préférence encore supérieure à 1,5 m/s.

L'invention concerne également une installation de production d'une composition de fructose comprenant :
- une ligne d'amenée de composition initiale comprenant du glucose ;
- un premier évaporateur alimenté par la ligne d'amenée de composition initiale ;
- une ligne de collecte de composition initiale concentrée en sortie du premier évaporateur ;
- un réacteur d'isomérisation du glucose en fructose alimenté par la ligne de collecte de composition initiale concentrée ;
- une ligne de collecte de composition intermédiaire en sortie du réacteur d'isomérisation ;
- un système de chromatographie multicolonnes, alimenté par la ligne de collecte de composition intermédiaire ;
- une ligne de collecte de raffinat et une ligne de collecte d'extrait en sortie du système de chromatographie multicolonnes ;
- un deuxième évaporateur alimenté par une ligne d'alimentation issue de la ligne de collecte d'extrait ;
- une ligne de collecte de composition de fructose en sortie du deuxième évaporateur ;
dans laquelle la ligne de collecte de composition intermédiaire n'alimente pas un évaporateur; et dans laquelle la ligne de collecte du raffinat est connectée à la ligne d'amenée de composition initiale de sorte à alimenter le premier évaporateur. U

Dans certains modes de réalisation, l'installation comprend une unité de décoloration de l'extrait située entre le système de chromatographie multicolonnes et le deuxième évaporateur, comprenant de préférence un lit de résine échangeuse d'ions et/ou de charbon actif, ainsi que, de préférence, une unité de filtration stérile.

Dans certains modes de réalisation, l'installation comprend une unité de déminéralisation entre le réacteur d'isomérisation et le système de chromatographie multicolonnes.

Dans certains modes de réalisation, la ligne de collecte de raffinat est connectée à la ligne d'amenée de composition initiale de sorte à alimenter le premier évaporateur.

Dans certains modes de réalisation, l'installation comprend une ligne de dérivation de composition intermédiaire issue de la ligne de collecte de composition intermédiaire et connectée à la ligne de collecte d'extrait de sorte à alimenter le deuxième évaporateur par la ligne d'alimentation.

Dans certains modes de réalisation, le système de chromatographie multicolonnes comprend de 4 à 6 cellules ; et/ou comprend des colonnes ayant une longueur de 1,0 à 2,6 m, de préférence de 1,4 à 2,0 m.

Dans certains modes de réalisation, le système de chromatographie multicolonnes comprend une pluralité de colonnes et des liaisons fluidiques inter-colonnes, et le volume des liaisons fluidiques inter-colonnes est inférieur à 10 %, de préférence inférieur à 5 % et de préférence inférieur à 3 % du volume des colonnes.

La présente invention permet de répondre au besoin exprimé dans l'état de la technique. Elle fournit plus particulièrement un procédé et une installation permettant la production d'une composition à base de fructose, telle qu'une composition HFS 55 ou HFS 95, de manière plus efficace.

Cela est accompli grâce à l'utilisation de deux étapes d'évaporation seulement, et grâce à la suppression de l'étape d'évaporation entre l'isomérisation et la purification chromatographique ; ou en maintenant une concentration massique en matière sèche sensiblement constante entre l'étape d'isomérisation et l'étape de purification chromatographique.

De manière surprenante, les présents inventeurs ont découvert que l'utilisation d'une charge en entrée de la purification chromatographique moins concentrée que dans l'état de la technique améliore l'efficacité du procédé. En particulier, l'invention permet de réduire le volume d'éluant consommé (et donc la consommation énergétique), et/ou de réduire le volume de phase stationnaire utilisé, et/ou d'augmenter le volume de charge à traiter, et/ou de réduire la taille des colonnes chromatographiques, et ce le cas échéant avec une installation de production plus compacte et moins onéreuse.

### BREVE DESCRIPTION DES FIGURES

La **figure 1** représente de manière schématique une installation comparative (qui n'est pas selon l'invention).
La **figure 2** représente de manière schématique un mode de réalisation d'une installation selon l'invention.
La **figure 3** représente de manière schématique un système chromatographique SSMB susceptible d'être utilisé pour mettre en oeuvre le procédé de l'invention.

### DESCRIPTION DE MODES DE REALISATION DE L'INVENTION

L'invention est maintenant décrite plus en détail et de façon non limitative dans la description qui suit.

### Procédé comparatif

On décrit tout d'abord pour plus de facilité un procédé comparatif de production de composition de fructose, par rapport auquel le procédé de l'invention constitue une amélioration.

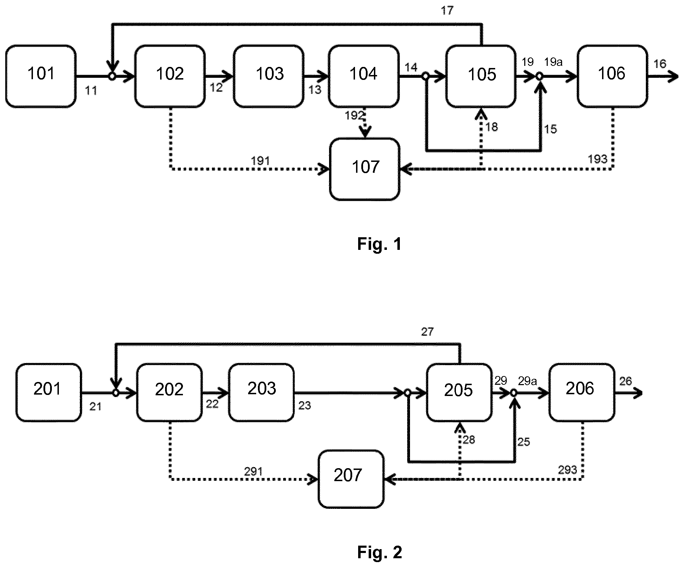

En faisant référence à la **figure 1****,** une installation pour la mise en oeuvre d'un procédé comparatif de production de composition de fructose comprend les éléments suivants :
- une source 101 de composition initiale comprenant du glucose ;
- un premier évaporateur 102 alimenté par une ligne d'amenée de composition initiale comprenant du glucose 11 issue de la source 101 de composition initiale comprenant du glucose ;
- une ligne de collecte de composition initiale concentrée 12 en sortie du premier évaporateur 102 ;
- un réacteur d'isomérisation 103 alimenté par la ligne de collecte de composition initiale concentrée 12 ;
- une ligne de collecte de composition intermédiaire 13 en sortie du réacteur d'isomérisation 103 ;
- un deuxième évaporateur 104 alimenté par la ligne de collecte de composition intermédiaire 13 ;
- une ligne de collecte de composition intermédiaire concentrée 14 en sortie du deuxième évaporateur 104 ;
- un système de chromatographie multicolonnes 105 alimenté par la ligne de collecte de composition intermédiaire concentrée 14 ainsi que par une ligne d'éluant 18 ;
- une ligne de collecte d'extrait 19 et une ligne de collecte de raffinat 17 issues du système de chromatographie multicolonnes 105, la ligne de collecte de raffinat 17 assurant un recyclage vers la ligne d'amenée de composition initiale 11 ;
- un troisième évaporateur 106 alimenté par une ligne d'alimentation 19a, elle-même alimentée par la ligne de collecte d'extrait 19 ;
- une ligne de collecte de composition de fructose 16 en sortie du troisième évaporateur 106 ;
- une première ligne de purge 191, une deuxième ligne de purge 192 et une troisième ligne de purge 193 en sortie respectivement du premier évaporateur 102, du deuxième évaporateur 104 et du troisième évaporateur 106 ; et
- une ligne de dérivation de composition intermédiaire concentrée 15, issue de la ligne de collecte de composition intermédiaire concentrée 14 et alimentant directement la ligne d'alimentation 19a (en combinaison avec la ligne de collecte d'extrait 19) en amont du troisième évaporateur 106. Alternativement, un ou plusieurs dispositifs intermédiaires peuvent être disposés sur la ligne 15. A titre d'exemple, un bac tampon peut être prévu pour le stockage de la composition intermédiaire.

Ainsi, selon le procédé comparatif, une composition initiale comprenant du glucose subit d'abord une étape de concentration dans le premier évaporateur 102, à l'issue de laquelle on récupère une composition initiale concentrée. Celle-ci est amenée vers le réacteur d'isomérisation 103, au sein duquel une partie du glucose est converti en fructose, dans une étape d'isomérisation. En sortie du réacteur d'isomérisation 103, on récupère une composition dite intermédiaire. Cette composition intermédiaire subit une étape de concentration dans le deuxième évaporateur 104, à l'issue de laquelle on récupère une composition intermédiaire concentrée. Celle-ci est amenée vers le système chromatographique multicolonnes 105, qui est séparément alimenté en éluant, à savoir de l'eau.

Une purification chromatographique est effectuée dans le système chromatographique multicolonnes 105, en sortie duquel on récupère un extrait et un raffinat. Le raffinat est enrichi en glucose par rapport à la composition intermédiaire, tandis que l'extrait est enrichi en fructose. Le raffinat est recyclé en le combinant avec la composition initiale avant la première étape de concentration. L'extrait est combiné avec une partie de la composition intermédiaire concentrée afin d'ajuster la concentration en fructose à une teneur souhaitée, puis ce flux est soumis à une étape de concentration dans le troisième évaporateur 106, à l'issue de laquelle on récupère la composition de fructose souhaitée.

De l'eau 107 est récupérée à partir de la première ligne de purge 191, de la deuxième ligne de purge 192 et de la troisième ligne de purge 193, et est utilisée comme source d'éluant pour le système chromatographique multicolonnes 105 ou comme toute autre source d'eau utilisable dans l'installation ou les opérations unitaires proches.

### Présentation générale du procédé de l'invention

Par rapport au procédé comparatif ci-dessus, l'invention prévoit avantageusement la suppression de l'étape de concentration entre l'isomérisation et la purification chromatographique, et donc la suppression du deuxième évaporateur. Le troisième évaporateur dans le procédé comparatif devient donc le deuxième évaporateur dans l'invention.

Ainsi, en faisant référence à la **figure 2****,** un exemple d'installation pour la mise en oeuvre du procédé de production de composition de fructose peut comprendre les éléments suivants :
- une source 201 de composition initiale comprenant du glucose ;
- un premier évaporateur 202 alimenté par une ligne d'amenée de composition initiale comprenant du glucose 21 issue de la source 201 de composition initiale comprenant du glucose ;
- une ligne de collecte de composition initiale concentrée 22 en sortie du premier évaporateur 202 ;
- un réacteur d'isomérisation 203 alimenté par la ligne de collecte de composition initiale concentrée 22 ;
- une ligne de collecte de composition intermédiaire 23 en sortie du réacteur d'isomérisation 203 ;
- un système de chromatographie multicolonnes 205 alimenté par la ligne de collecte de composition intermédiaire 23 ainsi que par une ligne d'éluant 28 ;
- une ligne de collecte d'extrait 29 et une ligne de collecte de raffinat 27 issues du système de chromatographie multicolonnes 5, la ligne de collecte de raffinat 27 assurant optionnellement un recyclage vers la ligne d'amenée de composition initiale 21 ;
- un deuxième évaporateur 206 alimenté par une ligne d'alimentation 29a, elle-même alimentée par la ligne de collecte d'extrait 29 ;
- une ligne de collecte de composition de fructose 26 en sortie du deuxième évaporateur 206 ;
- une première ligne de purge 291 et une deuxième ligne de purge 293 en sortie respectivement du premier évaporateur 202 et du deuxième évaporateur 206 ; et
- optionnellement, une ligne de dérivation de composition intermédiaire 25, issue de la ligne de collecte de composition intermédiaire 23 et alimentant directement la ligne d'alimentation 29a (en combinaison avec la ligne de collecte d'extrait 29) en amont du deuxième évaporateur 206. Alternativement, un ou plusieurs dispositifs intermédiaires peuvent être disposés sur la ligne 25. A titre d'exemple, un bac tampon peut être prévu pour le stockage de la composition intermédiaire.

Ainsi, selon le procédé de l'invention, une composition initiale comprenant du glucose subit d'abord une étape de concentration dans le premier évaporateur 202, à l'issue de laquelle on récupère une composition initiale concentrée. Celle-ci est amenée vers le réacteur d'isomérisation 203, au sein duquel une partie du glucose est converti en fructose, dans une étape d'isomérisation. L'isomérisation du glucose en fructose n'est pas totale. En sortie du réacteur d'isomérisation 203, on récupère une composition dite intermédiaire. Celle-ci est amenée vers le système chromatographique multicolonnes 205, qui est séparément alimenté en éluant, à savoir de l'eau.

Une purification chromatographique est effectuée dans le système chromatographique multicolonnes 205, en sortie duquel on récupère un extrait et un raffinat. Le raffinat est enrichi en glucose par rapport à la composition intermédiaire, tandis que l'extrait est enrichi en fructose. Le raffinat est recyclé en le combinant avec la composition initiale avant la première étape de concentration.

L'extrait peut être combiné avec une partie de la composition intermédiaire afin d'ajuster la concentration en fructose à une teneur souhaitée, puis ce flux est soumis à une étape de concentration dans le troisième évaporateur 206, à l'issue de laquelle on récupère la composition de fructose souhaitée. Cela est en particulier utile lorsqu'on souhaite obtenir une pureté finale en fructose peu élevée (par exemple, composition de type HFS 55).

Alternativement, et contrairement à ce qui est illustré sur la figure, l'extrait peut être directement soumis à l'étape de concentration dans le troisième évaporateur 206, à l'issue de laquelle on récupère la composition de fructose souhaitée, sans le combiner à un autre flux. Cela est en particulier utile lorsqu'on souhaite obtenir une pureté finale en fructose élevée (par exemple, composition de type HFS 95).

Dans le cadre de la production de fructose de haute pureté, la ligne de dérivation de composition intermédiaire 25 de la **figure 2** peut être supprimée et la ligne de collecte de raffinat 27 peut permettre un recyclage vers des unités de saccharification et/ou de déminéralisation en amont de la source 201. Afin d'éviter la concentration des polysaccharides du fait de ce recyclage, il peut être placé sur cette ligne de collecte de raffinat 27 une unité de nanofiltration ou une unité de séparation chromatographique par exemple, afin d'éliminer les polysaccharides.

Le rapport massique de la partie de composition intermédiaire qui est optionnellement prélevée (dans la ligne de dérivation de composition intermédiaire 25) (et optionnellement combinée avec l'extrait) par rapport à la composition intermédiaire totale (dans la ligne de collecte de composition intermédiaire 23) peut notamment valoir de 0,4 à 0,6, de préférence de 0,45 à 0,55, de préférence encore de 0,48 à 0,52, en particulier lorsque la teneur massique en fructose de la composition intermédiaire totale est proche de 42 %. Ce rapport massique peut notamment valoir de 0,45 à 0,65, de préférence de 0,50 à 0,65, de préférence encore de 0,57 à 0,61, en particulier lorsque la teneur massique en fructose de la composition intermédiaire totale est proche de 44 %.

De l'eau 207 est récupérée à partir de la première ligne de purge 291 et de la deuxième ligne de purge 293. Cette eau récupérée peut être utilisée comme source d'éluant pour le système chromatographique multicolonnes 205 ; alternativement, de l'eau fraîche peut être utilisée en tout ou partie pour l'éluant.

De préférence, une étape de déminéralisation (non représentée sur la figure) peut être effectuée entre l'étape d'isomérisation 203 et l'étape de chromatographie 205. Le système de déminéralisation comprend alors des colonnes remplies de résine échangeuse d'ions, cationique et/ou anionique, en tant que phase stationnaire.

De préférence, une étape d'élimination de la couleur résiduelle de l'extrait (ou décoloration) est effectuée avant la concentration de l'extrait dans le deuxième évaporateur 206. Celle-ci peut être effectuée en disposant une unité de décoloration (non représentée sur la figure) entre le système de chromatographie multicolonnes 205 et le deuxième évaporateur 206. La décoloration peut comprendre le passage du flux d'extrait sur une résine échangeuse d'ions cationique et/ou anionique et/ou sur un lit de charbon actif sous forme de poudre ou grains. Une filtration stérile peut être associée à cette étape.

Le premier évaporateur 202 et le deuxième évaporateur 206 peuvent être à plaques ou tubulaires, à simple effet ou multiples effets, à simple passe ou recirculation, à vapeur ou à recompression mécanique de vapeur, avec ou sans thermocompresseur.

La réaction d'isomérisation est de préférence une réaction enzymatique. Une enzyme telle que l'isomérase est mise en contact avec le produit dans un réacteur, de préférence à une température comprise entre 50 et 60°C, de préférence à un pH compris entre 7 et 8. Les enzymes tels que Sweetzyme^{®} de Novozymes ou Gensweet^{®} de Genencor sont utilisables pour cette opération.

Le procédé de l'invention est de préférence continu.

De préférence, la composition intermédiaire obtenue à l'issue de l'étape d'isomérisation ne subit pas d'étape de concentration par évaporation d'eau avant l'étape de purification chromatographique. Autrement, dit, aucun évaporateur n'est prévu entre le réacteur d'isomérisation 203 et le système de chromatographie multicolonnes 205.

Il est possible que la ligne de collecte de composition intermédiaire 23 relie directement le réacteur d'isomérisation 203 au système de chromatographie multicolonnes 205, sans aucun intermédiaire. Alternativement, un ou plusieurs dispositifs intermédiaires peuvent être disposés entre le réacteur d'isomérisation 203 et le système de chromatographie multicolonnes 205. A titre d'exemple, un bac tampon peut être prévu pour le stockage de la composition intermédiaire.

De préférence, la concentration massique en matière sèche de la composition intermédiaire en entrée du système de chromatographie multicolonnes 205 est égale à la concentration massique en matière sèche de la composition intermédiaire en sortie du réacteur d'isomérisation 203, le cas échéant à ± 5 % près, ou à ± 4 % près, ou à ± 3 % près, ou à ± 2 % près, ou à ± 1 % près, ou de manière stricte.

La tolérance ci-dessus est exprimée en pourcentages de matière sèche. Pour prendre un exemple, si une composition présente une concentration massique en matière sèche de 50 % à ± 5 % près, cela signifie que la composition présente une concentration massique en matière sèche de 45 à 55 %.

Il est possible d'envisager par exemple une légère dilution de la composition intermédiaire avant le système de chromatographie multicolonnes 205, par exemple par un apport d'eau. Mais il est préféré pour plus de simplicité qu'aucun ajustement actif de la concentration massique en matière sèche de la composition intermédiaire ne soit effectué entre le réacteur d'isomérisation 203 et le système de chromatographie multicolonnes 205.

### Compositions

La composition initiale utilisée dans le procédé de l'invention comprend du glucose. Il s'agit de préférence d'une composition aqueuse. Il s'agit de préférence d'un sirop de glucose dit à haut niveau de dextrose. Elle présente de préférence une concentration massique en matière sèche de 25 à 35 %, de préférence de 28 à 33 %, et de préférence encore d'environ 31 %.

De manière générale, la concentration massique en matière sèche d'une composition correspond à la masse de matière sèche de la composition rapportée à la masse totale de celle-ci. Dans les compositions utilisées dans l'invention, la concentration massique en matière sèche est approximativement égale à la teneur en sucres en degrés Brix.

La composition initiale utilisée dans le procédé de l'invention contient de préférence une proportion massique de glucose (par rapport à la matière sèche) supérieure ou égale à 50 %, ou à 80 %, ou à 90 %. De préférence encore, elle contient une proportion massique de glucose de 95 % environ. De préférence, le reste de la matière sèche est principalement composé de polysaccharides.

Après l'étape de concentration, on obtient la composition initiale concentrée. Celle-ci présente essentiellement la même composition en sucres que la composition initiale, mais elle présente une concentration massique en matière sèche supérieure, par exemple de 40 à 58 %, de préférence de 45 à 55 %, de préférence encore de 48 à 52 %, et de préférence encore d'environ 50 %.

La composition intermédiaire, qui est obtenue à l'issue de l'isomérisation, comporte également une concentration massique en matière sèche par exemple de 40 à 58 %, de préférence de 45 à 55 %, de préférence encore de 48 à 52 %, et de préférence encore d'environ 50 %. De préférence, la concentration massique en matière sèche de la composition intermédiaire à l'issue de l'isomérisation est sensiblement identique à la concentration massique en matière sèche de la composition initiale concentrée.

Lors de l'étape d'isomérisation, une partie du glucose est converti en fructose. Dans certains modes de réalisation, la composition intermédiaire contient une proportion massique de glucose (par rapport à la matière sèche) de 40 à 65 %, de préférence de 45 à 60 %, de préférence encore de 50 à 55 % et par exemple d'environ 53 %. Dans certains modes de réalisation, la composition intermédiaire contient une proportion massique de fructose (par rapport à la matière sèche) de 30 à 55 %, de préférence de 35 à 50 %, de préférence encore de 40 à 45 % et par exemple d'environ 42 %. Dans certains modes de réalisation, la composition intermédiaire contient une proportion massique de polysaccharides (par rapport à la matière sèche) de 1 à 10 %, de préférence de 3 à 8 %, de préférence encore de 4 à 6 % et par exemple d'environ 5 %.

Dans certains modes de réalisation, la composition intermédiaire contient une proportion massique de fructose de plus de 42 % et moins de 45 %, et par exemple une proportion massique de fructose d'environ 43 % ou d'environ 44 %. Il a en effet été constaté que le passage d'une proportion massique de fructose de 42 % à 43 % permet d'améliorer les indicateurs de consommation d'eau et de besoin en volume de résine de l'ordre de 7 à 9 %. La proportion massique de fructose dans la composition intermédiaire peut être ajustée en modifiant les conditions opératoires de la réaction d'isomérisation (durée par exemple).

A l'issue de la purification chromatographique, on obtient un extrait enrichi en fructose et donc appauvri en glucose et un raffinat enrichi en glucose et donc appauvri en fructose.

Par fraction « *enrichie* » en une espèce A et « appauvrie » en une espèce B, on entend que le rapport de concentrations molaires espèce A / espèce B dans la fraction est supérieur à celui du flux en entrée de la purification chromatographique (indépendamment des effets de concentration ou de dilution globale).

Dans certains modes de réalisation, la composition de fructose récupérée (obtenue après concentration de l'extrait, dans la ligne de collecte de composition de fructose 26) comporte une concentration massique en matière sèche d'au moins 75 %, de préférence d'au moins 76 %, et par exemple d'environ 77 %.

Dans certains modes de réalisation, la composition de fructose récupérée contient une proportion massique de glucose (par rapport à la matière sèche) de 35 à 48 %, de préférence de 38 à 45 %, de préférence encore de 39 à 42 %, par exemple d'environ 40 %. Dans certains modes de réalisation, la composition de fructose récupérée contient une proportion massique de fructose (par rapport à la matière sèche) de 50 à 60 %, de préférence de 52 à 58 %, de préférence encore de 54 à 56 %, par exemple d'environ 55 %. Dans certains modes de réalisation, la composition de fructose récupérée contient une proportion massique de polysaccharides (par rapport à la matière sèche) de 2 à 8 %, de préférence de 3 à 7 %, de préférence encore de 4 à 6 %, par exemple d'environ 5 %.

Dans d'autres modes de réalisation, la composition de fructose récupérée contient une proportion massique de fructose (par rapport à la matière sèche) supérieure ou égale à 95 %, de préférence à 96 %, de préférence encore à 97 %, de préférence encore à 98 %, de préférence encore à 98,5 %.

De préférence, au moins 80 % en masse du fructose contenu dans le mélange à séparer est récupéré dans l'extrait, de préférence encore au moins 90 % en masse.

### Purification chromatographique

La purification chromatographique est effectuée dans un ensemble de plusieurs colonnes de chromatographie contenant une phase stationnaire, avec successivement, de manière cyclique, dans une partie donnée du système :
- une étape de collecte d'un raffinat, une étape d'injection du mélange à séparer, une étape de collecte d'un extrait et une étape d'injection de phase mobile.

Les différentes étapes ci-dessus se succèdent temporellement dans une partie du système. La partie en question du système est de préférence située entre la sortie d'une colonne et l'entrée de la colonne suivante. Alternativement, la partie en question du système peut inclure une colonne ou une partie de colonne.

A un instant donné, une ou plusieurs des étapes ci-dessus peuvent être simultanément mises en oeuvre dans une ou plusieurs parties du système. Par exemple, toutes ces étapes peuvent être simultanément mises en oeuvre dans des parties respectives du système.

Le « *mélange à séparer »,* ou « *charge* », ou « *charge à traiter »* est le mélange contenant un produit d'intérêt (ici le fructose) qui est soumis à la purification chromatographique. La purification a vocation à enrichir une fraction (ici l'extrait) en ledit produit d'intérêt.

Par « *raffinat »,* on entend la fraction obtenue par élution qui contient les espèces relativement les moins retenues par la phase stationnaire, et donc dont l'élution est la plus rapide.

Par « *extrait »,* on entend la fraction obtenue par élution qui contient les espèces relativement les plus retenues par la phase stationnaire, et donc dont l'élution est la plus lente.

L'éluant est un fluide injecté pour déplacer les espèces retenues par la phase stationnaire. Dans l'invention, l'éluant utilisé est de préférence de l'eau.

Par « *phase mobile »* on entend le fluide qui se déplace dans les colonnes du système. Selon sa position, chaque colonne est traversée par un volume de phase mobile dépendant de la zone dans laquelle se trouve la colonne, ce volume pouvant être différent du volume d'éluant qui est injecté dans l'une ou l'autre des colonnes. Dans le cas d'un procédé multicolonnes avec des zones identifiées entre les lignes d'entrées et sorties (comme décrit plus en détail ci-dessous), le terme « *volume de phase mobile »* désigne le volume de fluide qui entre dans une zone. Ce fluide peut être différent de l'éluant au sens strict, mais il contribue au déplacement des produits dans chaque colonne de la zone. On parle ainsi de volume de phase mobile associé à chaque zone. La configuration préférée des zones dans le système chromatographique est décrite plus en détail ci-après.

Dans certains modes de réalisation avantageux, le système chromatographique comprend des organes de séquençage des lignes d'injection et de collecte. En particulier, le séquençage de ces lignes d'injection et de collecte a lieu sur un cycle de fonctionnement du système. Dans la présente demande, un *« cycle de fonctionnement* » ou « *cycle »* désigne la durée au bout de laquelle les lignes d'injection et de collecte ont été séquencées jusqu'à revenir à leur position initiale dans le système. Au bout d'un cycle, le système est à nouveau dans sa configuration initiale. Un cycle comporte en général autant de « *périodes* » que de colonnes. Ainsi le cycle d'un procédé mis en oeuvre sur un système à 8 colonnes est composé de 8 périodes successives.

L'unité BV (« *Bed Volume* ») permet de mesurer le volume de phase mobile circulant dans chaque zone (ou d'éluant injecté, ou de charge à traiter injectée), rapporté au volume de lit de phase stationnaire dans une colonne. La mesure de ces volumes s'entend par période.

La phase stationnaire utilisée dans l'invention est de préférence une résine cationique forte divalente sous forme calcium, ayant une granulométrie (Dv50) comprise entre 100 et 600 µm , de préférence comprise entre 170 et 400 µm.

La purification chromatographique de l'invention est mise en oeuvre dans un système chromatographique multicolonnes. De préférence, le système chromatographique comprend de 4 à 10 colonnes.

De préférence, la purification chromatographique de l'invention de l'invention est mise en oeuvre de façon continue.

De préférence, la purification chromatographique de l'invention est un procédé chromatographique périodique à accumulation.

Par « *procédé à accumulation* », on entend un procédé chromatographique dans lequel l'injection du mélange à séparer (flux de départ) est intercalée ou additionnée à un profil de concentration non nulle passant de la sortie à l'entrée d'une colonne.

Des exemples de tels procédés à accumulation sont les procédés AMB, SMB, VariCol, Powerfeed, ModiCon, iSMB ou SSMB.

Le procédé à lit mobile simulé (ou SMB, pour « *simulated moving bed*») est un procédé multicolonnes continu, l'injection de mélange à séparer étant effectuée sur l'ensemble d'un cycle.

Le procédé SMB peut être notamment un procédé SMB à quatre zones. Dans ce cas, le système comporte un ensemble de colonnes montées en série et en boucle fermée, la sortie d'une colonne étant reliée à une entrée de colonne suivante. Le système comprend au moins une ligne d'injection de mélange à séparer, une ligne de collecte de raffinat, une ligne d'injection d'éluant et une ligne de collecte d'extrait. Les lignes d'injection (de flux de départ et d'éluant) et les lignes de collecte de fractions se déplacent périodiquement et de manière synchrone (séquençage synchrone) au sein de la boucle dans la direction de l'écoulement du fluide circulant à travers la boucle. La durée entre deux décalages de l'ensemble des lignes d'injection et de collecte d'une colonne correspond à une période ; au bout d'un cycle tous les points sont revenus à leur position initiale, le système ayant un fonctionnement cyclique. Un cycle comporte autant de périodes que de colonnes.

Un système AMB (lit mobile réel, ou « *actual moving bed*») présente un fonctionnement similaire à un système SMB. Toutefois, au lieu de déplacer les points d'injection du flux d'alimentation et de l'éluant, ainsi que des points de collecte, au moyen d'un système de vannes, un ensemble d'unités d'adsorption (colonnes) sont déplacées physiquement par rapport aux points d'alimentation et de collecte. A nouveau, le fonctionnement permet de simuler un lit mobile continu à contre-courant.

La purification chromatographique de l'invention peut être un procédé à injection continue du mélange à séparer (c'est-à-dire un procédé dans lequel l'injection du mélange à séparer est un flux continu). L'injection du mélange à séparer est alors effectuée pendant toute la durée du cycle. La purification chromatographique de l'invention peut également être un procédé à injection quasi-continue du mélange à séparer.

Alternativement, la purification chromatographique de l'invention peut être un procédé dans lequel l'injection du mélange à séparer (flux de départ) est discontinue. Dans ces procédés, l'injection du mélange à séparer n'est pas faite sur l'ensemble d'un cycle mais pendant une durée totale inférieure à un cycle. On peut citer comme procédé à injection discontinue de mélange à séparer le procédé iSMB (« *improved simulated moving bed* » ou SMB amélioré en français), décrit dans les documents EP 0342629 et US 5,064,539, auxquels il est fait expressément référence. Dans ce procédé, dans une étape le système fonctionne en boucle fermée, sans injection ou collecte de produit.

Le procédé SMB séquentiel, ou SSMB (« *sequential simulated moving bed* ») est un autre exemple préféré. Un système SSMB découpe les introductions et collectes des flux en sous séquences appliquées de façons périodiques. Un système SSMB est par exemple décrit dans le document WO 2015/104464.

De préférence, la purification chromatographique de l'invention est un procédé de type SSMB.

Le système chromatographique comprend de préférence des zones 1, 2, 3 et 4 : la zone 1 est située entre une ligne d'injection d'éluant et une ligne de collecte de l'extrait ; la zone 2 est située entre la ligne de collecte de l'extrait et une ligne d'injection du mélange à séparer ; la zone 3 est située entre la ligne d'injection du mélange à séparer et une ligne de collecte du raffinat ; et la zone 4 est située entre ladite ligne de collecte du raffinat et la ligne d'injection d'éluant.

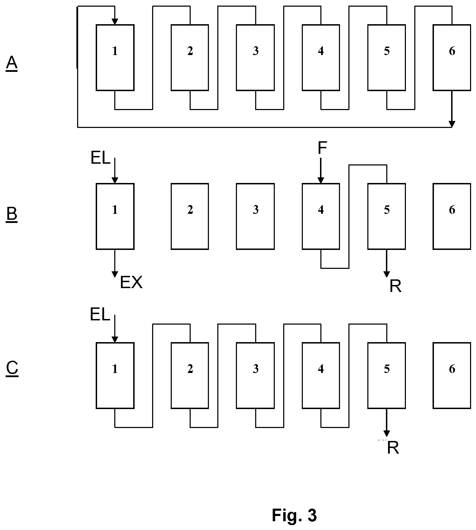

Un exemple possible de système SSMB utilisable dans l'invention est représenté en référence à la **figure 3****.** Dans cet exemple, on utilise six cellules ou colonnes. Ce système peut être opéré selon un fonctionnement cyclique en quatre phases.
- Phase n°1 (partie A de la figure) : phase de boucle, durant laquelle on maintient une circulation continue en boucle fermée sur toutes les cellules placées en série, pour déplacer le volume interstitiel d'une cellule vers la suivante, sans injection d'éluant. L'homme de l'art observe que le volume de phase mobile déplacé dans cette phase contribue aux zones 1, 2, 3 et 4.
- Phase n°2 (partie B de la figure) : charge / injection de charge. La charge du flux (F) est injectée en tête de la quatrième cellule. Simultanément, on collecte un volume sensiblement identique de raffinat (R) en sortie de la cinquième cellule. Les cellules 4 et 5 constituent ici la zone 3. Les cellules 2 et 3 constituent la zone de séparation entre extrait et injection de charge. Elles constituent ici la zone 2. L'homme de l'art observe que le volume de phase mobile déplacé dans cette phase contribue à la zone 3.
- Phase n°3 (partie B de la figure) : élution de l'extrait. L'éluant (EL) est injecté sur la première cellule pour éluer l'extrait (EX), qui est collecté en volume sensiblement identique au bas de la première cellule. La cellule n°1 constitue ici la zone 1. L'homme de l'art observe que le volume de phase mobile déplacé dans cette phase contribue à la zone 1.

- Les phases n°2 et 3 sont de préférence opérées simultanément pour accroître la productivité du système.
- Phase n°4 (partie C de la figure) : élution du raffinat. L'éluant (EL) est injecté en tête de la première cellule, et le raffinat (R) est collecté en volume sensiblement identique sortie de la cinquième. La cellule n°6 est ici une cellule tampon permettant d'assurer la séparation entre la queue de l'extrait et la tête du raffinat. Elle constitue la zone 4. Cette zone peut être omise dans le cas où le degré de pureté et/ou le rendement recherché est relativement limité. L'homme de l'art observe que le volume de phase mobile déplacé dans cette phase contribue aux zones 1, 2 et 3.

Ces phases sont opérées dans l'ordre dans un mode de réalisation préféré, de 1 à 4. Leur enchaînement constitue une séquence complète (également appelée période).

Chaque séquence (phases n°1 à 4) est répétée six fois en décalant les entrées et sorties de cellules par incrémentation du numéro de cellule, de la gauche vers la droite du système : la charge est ainsi injectée en haut de cellule n°1 en séquence n°1, puis en haut de cellule n°2 en séquence n°2, etc.

Un cycle de production complet est réalisé après achèvement des six séquences successives, quand le point d'injection de la charge, initialement en entrée de cellule n°1, revient de nouveau en entrée de cellule n°1.

Dans ce qui précède, on a donné une description du système SSMB en référence au cas où les cellules correspondent à des colonnes. Ceci n'est pas limitatif, et l'invention s'applique aussi aux systèmes dans lesquels les cellules, ou encore compartiments, sont des parties de colonne.

Par ailleurs, le nombre de colonnes présentes en zones 1, 2, 3 et 4 peut varier en fonction de la qualité de séparation désirée. On peut donc concevoir des systèmes du même type avec une cellule, deux cellules, trois cellules, quatre cellules, cinq cellules, six cellules, et jusqu'à douze cellules ou plus.

Les colonnes peuvent avoir notamment une longueur de 1 à 2,6 m, à savoir : de 1,0 à 1,2 m, ou de 1,2 à 1,4 m, ou de 1,4 à 1,6 m, ou de 1,6 à 1,8 m, ou de 1,8 à 2,0 m, ou de 2,0 à 2,2 m, ou de 2,2 à 2,4 m, ou de 2,4 à 2,6 m ; une gamme de 1,4 à 2,0 m est considérée comme préférée. La longueur en question est la longueur utile de la colonne, correspondant à la hauteur du lit de phase stationnaire dans la colonne.

Comme décrit ci-dessus et comme illustré dans les exemples ci-dessous, l'invention permet d'améliorer la performance de l'installation chromatographique. Toutefois, des pertes de performances peuvent être rencontrées lors d'un changement d'échelle de l'installation. En particulier, lorsque le diamètre des colonnes dépasse environ un mètre de diamètre, il peut être crucial de maitriser les volumes morts.

Les volumes morts correspondent au volume (interne) total des « *liaisons fluidiques inter-colonnes* », c'est-à-dire des liaisons entre la ou les sorties d'une colonne et la ou les entrées de la colonne suivante. Tout élément se trouvant entre deux colonnes successives, tel qu'un tuyau (ou conduite), une vanne ou une pompe, appartient aux liaisons fluidiques inter-colonnes. Ne sont pas considérés comme relevant des volumes morts les volumes se trouvant après une vanne de collecte d'extrait ou de raffinat, ou avant des vannes d'injection de charge ou d'éluant (volumes situés entre le système de chromatographie et les réservoirs de charge à injecter, éluant, extrait et raffinat).

Il est avantageux que la vitesse des fluides circulant dans les liaisons fluidiques inter-colonnes, et en particulier dans les tuyaux ou conduites de celles-ci, dépasse 0,5 m/s, préférentiellement 1 m/s et de préférence encore 1,5 m/s. La vitesse des fluides considérée ici est la vitesse moyenne (débit divisé par la section transversale).

Le contrôle de la vitesse dans les liaisons fluidiques inter-colonnes est effectué par exemple en ajustant le diamètre de ces liaisons pour un débit considéré.

Il est également avantageux que le volume total des liaisons fluidiques inter-colonnes soit inférieur à 10 % du volume total des colonnes, de préférence inférieur à 5 %, voire à 3 % du volume total des colonnes. Cela permet d'éviter une baisse des performances pouvant atteindre un à deux points de pureté ou de rendement. L'ajustement de ces volumes morts peut être effectué en minimisant la longueur totale des liaisons fluidiques inter-colonnes (notamment tuyaux ou conduites).

De préférence, les valeurs de vitesse de fluides et de volumes morts ci-dessus sont associées à des colonnes ayant un diamètre supérieur ou égal à 1 m (diamètre utile, ou diamètre du lit de phase stationnaire dans les colonnes).

### Réglage de la purification chromatographique

Les débits de fluide dans les différentes colonnes du système chromatographique peuvent être ajustés de sorte à obtenir les paramètres de fonctionnement suivants.

Le rapport du débit massique d'éluant sur le débit massique de matière sèche de composition de fructose produite peut valoir de 0,5 à 0,6 ; ou de 0,6 à 0,7 ; ou de 0,7 à 0,8 ; ou de 0,8 à 0,9 ; ou de 0,9 à 1,0 ; ou de 1,0 à 1,1 ; ou de 1,1 à 1,2 ; ou de 1,2 à 1,3. Des gammes de 0,5 à 1,3, notamment 0,6 à 1,2, sont des exemples de gammes préférées.

Le volume d'éluant injecté peut être notamment de 0,12 à 0,14 BV ; ou de 0,14 à 0,16 BV ; ou de 0,16 à 0,18 BV ; ou de 0,18 à 0,20 BV ; ou de 0,20 à 0,22 BV ; ou de 0,22 à 0,24 BV.

Le volume de charge à traiter (volume de composition intermédiaire soumise à l'étape de purification chromatographique) peut être notamment de 0,13 à 0,16 BV ; ou de 0,16 à 0,18 BV ; ou de 0,18 à 0,20 BV ; ou de 0,20 à 0,22 BV ; ou de 0,22 à 0,24 BV ; ou de 0,24 à 0,26 BV ; ou de 0,26 à 0,28 BV ; ou de 0,28 à 0,30 BV ; ou de 0,30 à 0,32 BV ; ou de 0,32 à 0,34 BV ; ou de 0,34 à 0,36 BV ; ou de 0,36 à 0,38 BV ; ou de 0,38 à 0,40 BV.

La purification chromatographique est de préférence effectuée à une température supérieure ou égale à 50°C ; et notamment : de 50 à 53°C ; ou de 53 à 55°C ; ou de 55 à 58°C, ou de 58 à 60°C ; ou de 60 à 62°C ; ou de 62 à 65°C ; ou de 65 à 70°C. Une température de 60°C environ est un exemple de température particulièrement appropriée. La température ci-dessus correspond à la température moyenne à laquelle se trouve la phase mobile dans le système chromatographique.

### EXEMPLES

Les exemples suivants illustrent l'invention sans la limiter. Dans l'ensemble des exemples, un système de purification chromatographique de type SSMB a été utilisé. Le système comprend 4 colonnes remplies de résine XA2004-30Ca ou XA2004-31Ca de Novasep Process en tant que phase stationnaire, sur une hauteur de lit de deux mètres dans chaque colonne.

On note BV₁, BV₂, BV₃ et BV₄ les volumes respectifs de phase mobile dans les zones 1, 2, 3 et 4. Dans les exemples ci-dessous, le volume d'éluant (noté BVₑₐᵤ et égal à BV₁-BV₄) est ajusté dans une plage de 0,11 à 0,25. Le volume de charge à traiter (noté BV_{feed} et égal à BV₃-BV₂) est ajusté dans une plage de 0,11 à 0,30. On effectue un balayage des débits BV₁ et BV₂ sur les plages respectives suivantes : de 0,65 à 0,75 et de 0,55 à 0,65. Les volumes BV₃ et BV₄ sont calculés de la façon suivante : BV₃=BV₂+BVf_{eed} et BV₄=BV₁-BVₑₐᵤ.

Les ajustements de BV₁ et BV₂ fluctuent d'un système à l'autre pour des raisons de variabilité de densité de phase stationnaire. En revanche, cette variabilité n'a pas d'impact sur les performances dues au volume d'eau et au volume de charge utilisés.

Pour chaque réglage effectué, des puretés et des rendements sont mesurés expérimentalement, mais les puretés et les rendements ne sont pas en eux-mêmes importants pour caractériser l'invention. En effet, quelle que soit la pureté en fructose de l'extrait obtenu en sortie de chromatographie, un mélange partiel avec le mélange non enrichi est effectué avant la dernière évaporation.

Pour des raisons de clarté, les exemples suivants présentent directement les résultats de bilan matière correspondants aux meilleurs réglages obtenus. Les tableaux présentés correspondent à une production journalière de 250 tonnes de composition de fructose HFS 55 en matière sèche, ce qui correspond à 310 tonnes de HFS 55 liquide à 77 % de matière sèche. Les performances de la chromatographie sont évaluées selon les rapports tₑₐᵤ/t_{HFS} (le rapport massique du débit journalier d'eau utilisée sur le débit journalier en matière sèche de composition de fructose produite) et V_{CHR}/t_{HFS} (le rapport du volume de phase stationnaire dans l'installation ramené à la masse de matière sèche de composition de fructose produite par journée).

### Exemple 1 (comparatif)

Cet exemple de référence est mis en oeuvre selon le schéma de la **figure 1** décrit ci-dessus (installation à trois évaporateurs, et flux en entrée de l'unité chromatographique présentant une concentration massique en matière sèche de 60 %).

Dans cet exemple, le volume d'eau (éluant) utilisé (BVₑₐᵤ) est égal à 0,177 BV et le volume de charge à traiter (BV_{feed}) est égal à 0,2 BV. Le tableau suivant résume les caractéristiques des compositions passant dans différentes lignes de l'installation. Le débit est indiqué en tonnes métriques par jour. Le taux de matière sèche est indiqué en pourcentage massique par rapport à la masse totale de la composition concernée. Les taux de fructose, de glucose et de polysaccharides sont indiqués en pourcentages massiques par rapport à la matière sèche de la composition concernée.

| Ligne | 11 | 12 | 13 | 14 | 15 | 16 | 18 | 193 |
|---|---|---|---|---|---|---|---|---|
| Débit | 807 | 694 | 694 | 578 | 296 | 326 | 194 | 326 |
| Matière sèche | 31% | 50% | 50% | 60% | 60% | 77% | 0 | 0 |
| Fructose | 0% | 2% | 42% | 42% | 42% | 55% | - | - |
| Glucose | 95% | 92% | 52% | 52% | 52% | 40% | - | - |
| Polysaccharides | 5% | 6% | 6% | 6% | 6% | 5% | - | - |

Dans cet exemple, le rapport tₑₐᵤ/t_{HFS} est égal à 0,78. Le rapport V_{CHR}/t_{HFS} est égal à 0,35m³/t.

### Exemple 2 (invention)

Cet exemple est mis en oeuvre selon le schéma de la **figure 2** décrit ci-dessus (installation à deux évaporateurs, et flux en entrée de l'unité chromatographique présentant une concentration massique en matière sèche de 50 %).

Dans cet exemple, le volume d'eau (éluant) utilisé est égal à 0,15 BV et le volume de charge à traiter est égal à 0,25 BV. Le tableau suivant résume les caractéristiques des compositions passant dans différentes lignes de l'installation, de la même manière que dans l'exemple 1.

| Ligne | 21 | 22 | 23 | 25 | 26 | 28 | 293 |
|---|---|---|---|---|---|---|---|
| Débit | 807 | 788 | 788 | 358 | 326 | 194 | 326 |
| Matière sèche | 31% | 50% | 50% | 50% | 77% | 0 | 0 |
| Fructose | 0% | 7% | 42% | 42% | 55% | - | - |
| Glucose | 95% | 88% | 53% | 53% | 40% | - | - |
| Polysaccharides | 5% | 5% | 5% | 5% | 5% | - | - |

Dans cet exemple, le rapport tₑₐᵤ/t_{HFS} est égal à 0,77. Le rapport V_{CHR}/t_{HFS} est égal à 0,3 m³/t.

En comparaison de l'exemple 1, il apparaît que malgré une baisse de concentration du produit en entrée de la chromatographie (de 60 à 50 % de matière sèche), tout en baissant le volume total d'eau d'élution et en augmentant celui de la charge à traiter, le volume de résine par tonne de produit final est plus faible.

### Exemple 3 (invention)

Cet exemple est similaire à l'exemple 2, à ceci près que le volume d'eau utilisé est égal à 0,14 BV et que le volume de charge à traiter est égal à 0,27 BV. Le tableau suivant résume les caractéristiques des compositions passant dans différentes lignes de l'installation, de la même manière que dans les exemples précédents :

| Ligne | 21 | 22 | 23 | 25 | 26 | 28 | 293 |
|---|---|---|---|---|---|---|---|
| Débit | 807 | 782 | 782 | 358 | 326 | 178 | 326 |
| Matière sèche | 31% | 50% | 50% | 50% | 77% | 0 | 0 |
| Fructose | 0% | 7% | 42% | 42% | 55% | - | - |
| Glucose | 95% | 88% | 53% | 53% | 40% | - | - |
| Polysaccharides | 5% | 5% | 5% | 5% | 5% | - | - |

Dans cet exemple, le rapport tₑₐᵤ/t_{HFS} est égal à 0,71. Le rapport V_{CHR}/t_{HFS} est égal à 0,32 m³/t. Cet exemple démontre que si l'on abaisse davantage le volume d'eau d'élution, tout en augmentant également le volume de charge à traiter (par rapport à l'exemple 2), le volume de résine par tonne de produit final est quelque peu supérieur mais la performance obtenue sur le volume d'eau par tonne de produit final reste très avantageuse.

### Exemple 4 (invention)

Cet exemple est similaire à l'exemple 2, à ceci près que le volume d'eau utilisé est égal à 0,14 BV et que le volume de charge à traiter est égal à 0,17. En outre, la longueur de colonnes est réduite à 1 m 40 au lieu de 2 m dans les exemples précédents. Le tableau suivant résume les caractéristiques des compositions passant dans différentes lignes de l'installation, de la même manière que dans les exemples précédents :

| Ligne | 21 | 22 | 23 | 25 | 26 | 28 | 293 |
|---|---|---|---|---|---|---|---|
| Débit | 807 | 700 | 700 | 341 | 326 | 264 | 326 |
| Matière sèche | 31% | 50% | 50% | 50% | 77% | 0 | 0 |
| Fructose | 0% | 3% | 42% | 42% | 55% | - | - |
| Glucose | 95% | 91% | 52% | 52% | 40% | - | - |
| Polysaccharides | 5% | 6% | 6% | 6% | 5% | - | - |

Dans cet exemple, le rapport tₑₐᵤ/t_{HFS} est égal à 0,96. Le rapport V_{CHR}/t_{HFS} est égal à 0,27 m³/t. Cet exemple démontre qu'il est possible de travailler avec des colonnes plus courtes tout en gardant une bonne productivité, ce qui permet d'obtenir des performances bonnes en consommation d'eau et particulièrement optimisées sur les besoins en résine. D'autres réglages peuvent être trouvés permettant de baisser la consommation d'eau pour des volumes de résines plus importants.

## Revendications

1. Procédé de production d'une composition de fructose comprenant les étapes successives suivantes :
- fourniture d'une composition initiale comprenant du glucose ;
- concentration de la composition initiale par évaporation d'eau pour obtenir une composition initiale concentrée ;
- isomérisation du glucose en fructose à partir de la composition initiale concentrée, permettant d'obtenir une composition intermédiaire ;
- purification de la composition intermédiaire dans un système de chromatographie multicolonnes, permettant d'obtenir un raffinat riche en glucose et un extrait riche en fructose ;
- concentration de l'extrait par évaporation d'eau ;
la composition intermédiaire n'étant pas soumise à une étape de concentration par évaporation d'eau entre l'étape d'isomérisation et l'étape de purification ; et
le raffinat étant recyclé et ajouté à la composition initiale avant l'étape de concentration de la composition initiale.

2. Procédé selon la revendication 1, comprenant en outre une étape d'élimination de couleur résiduelle de l'extrait avant l'étape de concentration de l'extrait, de préférence par résine échangeuse d'ions et/ou par charbon actif ainsi que, de préférence, une étape de filtration stérile.

3. Procédé selon la revendication 1 ou 2, comprenant en outre une étape de déminéralisation entre l'étape d'isomérisation et l'étape de chromatographie.

4. Procédé selon l'une des revendications 1 à 3, dans lequel la purification est effectuée à une température supérieure ou égale à 50°C, de préférence de 55 à 65°C, et de préférence encore d'environ 60°C.

5. Procédé selon l'une des revendications 1 à 4, dans lequel la composition intermédiaire qui est soumise à l'étape de purification présente une concentration massique en matière sèche de 45 à 55 %, et de préférence d'environ 50 %.

6. Procédé selon l'une des revendications 1 à 5, dans lequel la concentration massique en matière sèche de la composition intermédiaire varie de moins de 5%, et de préférence reste essentiellement constante, entre la fin de l'étape d'isomérisation et le début de l'étape de purification.

7. Procédé selon l'une des revendications 1 à 6, dans lequel le système de chromatographie multicolonnes comprend une pluralité de colonnes et des liaisons fluidiques inter-colonnes, et dans lequel la vitesse des fluides dans les liaisons fluidiques inter-colonnes est supérieure à 0,5 m/s, de préférence supérieure à 1 m/s et de préférence encore supérieure à 1,5 m/s.

8. Installation de production d'une composition de fructose comprenant :
- une ligne d'amenée de composition initiale (21) comprenant du glucose ;
- un premier évaporateur (202) alimenté par la ligne d'amenée de composition initiale (21);
- une ligne de collecte de composition initiale concentrée (22) en sortie du premier évaporateur (202) ;
- un réacteur d'isomérisation (203) du glucose en fructose alimenté par la ligne de collecte de composition initiale concentrée (22) ;
- une ligne de collecte de composition intermédiaire (23) en sortie du réacteur d'isomérisation (203) ;
- un système de chromatographie multicolonnes (205), alimenté par la ligne de collecte de composition intermédiaire (23) ;
- une ligne de collecte de raffinat (27) et une ligne de collecte d'extrait (29) en sortie du système de chromatographie multicolonnes (205) ;
- un deuxième évaporateur (206) alimenté par une ligne d'alimentation (29a) issue de la ligne de collecte d'extrait (29);
- une ligne de collecte de composition de fructose (26) en sortie du deuxième évaporateur (206) ;
dans laquelle la ligne de collecte de composition intermédiaire (23) n'alimente pas un évaporateur ; et
dans laquelle la ligne de collecte de raffinat (27) est connectée à la ligne d'amenée de composition initiale (21) de sorte à alimenter le premier évaporateur (202).

9. Installation selon la revendication 8, comprenant une unité de décoloration de l'extrait située entre le système de chromatographie multicolonnes (205) et le deuxième évaporateur (206), comprenant de préférence un lit de résine échangeuse d'ions et/ou de charbon actif, ainsi que, de préférence, une unité de filtration stérile.

10. Installation selon la revendication 8 ou 9, comprenant une unité de déminéralisation entre le réacteur d'isomérisation (203) et le système de chromatographie multicolonnes (205).

11. Installation selon l'une des revendications 8 à 10, comprenant une ligne de dérivation de composition intermédiaire (25) issue de la ligne de collecte de composition intermédiaire (23) et connectée à la ligne de collecte d'extrait (29) de sorte à alimenter le deuxième évaporateur (206) par la ligne d'alimentation (29a).

12. Installation selon l'une des revendications 8 à 11, dans laquelle le système de chromatographie multicolonnes (205) comprend de 4 à 6 cellules ; et/ou comprend des colonnes ayant une longueur de 1,0 à 2,6 m, de préférence de 1,4 à 2,0 m.

13. Installation selon l'une des revendications 8 à 12, dans laquelle le système de chromatographie multicolonnes (205) comprend une pluralité de colonnes et des liaisons fluidiques inter-colonnes, et dans laquelle le volume des liaisons fluidiques inter-colonnes est inférieur à 10 %, de préférence inférieur à 5 % et de préférence inférieur à 3 % du volume des colonnes.

## Patentansprüche

1. Herstellungsverfahren einer Fruktosezusammensetzung, umfassend die folgenden sukzessiven Schritte:
- Bereitstellen einer anfänglichen Zusammensetzung, umfassend Glukose;
- Konzentrieren der anfänglichen Zusammensetzung durch Verdampfen von Wasser, um eine konzentrierte anfängliche Zusammensetzung zu erhalten;
- Isomerisieren der Glukose zu Fruktose, ausgehend von der konzentrierten anfänglichen Zusammensetzung, wodurch ermöglicht wird, eine Zwischenzusammensetzung zu erhalten;
- Reinigen der Zwischenzusammensetzung in einem System der Mehrsäulenchromatographie, wodurch ermöglicht wird, ein glukosereiches Raffinat und einen fruktosereichen Extrakt zu erhalten;
- Konzentrieren des Extrakts durch Verdampfen von Wasser;
wobei die Zwischenzusammensetzung keinem Schritt des Konzentrierens durch Verdampfen von Wasser zwischen dem Schritt des Isomerisierens und dem Schritt des Reinigens unterzogen wird; und
wobei das Raffinat recycelt und der anfänglichen Zusammensetzung vor dem Schritt des Konzentrierens der anfänglichen Zusammensetzung zugegeben wird.

2. Verfahren nach Anspruch 1, umfassend außerdem einen Schritt des Eliminierens von restlicher Farbe des Extrakts vor dem Schritt des Konzentrierens des Extrakts, vorzugsweise durch Ionenaustauschharz und/oder durch Aktivkohle sowie vorzugsweise einen Schritt des sterilen Filtrierens.

3. Verfahren nach Anspruch 1 oder 2, umfassend außerdem einen Schritt des Demineralisierens zwischen dem Schritt des Isomerisierens und den Schritt des Chromatographie.

4. Verfahren nach einem der Ansprüche 1 bis 3, wobei das Reinigen bei einer Temperatur durchgeführt wird, die höher als oder gleich wie 50 °C, vorzugsweise von 55 bis 65 °C und noch bevorzugter ungefähr 60 °C ist.

5. Verfahren nach einem der Ansprüche 1 bis 4, wobei die Zwischenzusammensetzung, die dem Schritt des Reinigens unterzogen wird, eine Massenkonzentration an Trockensubstanz von 45 bis 55 %, und vorzugsweise ungefähr 50 % aufweist.

6. Verfahren nach einem der Ansprüche 1 bis 5, wobei die Massenkonzentration an Trockensubstanz die Zwischenzusammensetzung zwischen dem Ende des Schritts des Isomerisierens und dem Beginn des Schritts des Reinigens um weniger als 5 % variiert und vorzugsweise im Wesentlichen konstant bleibt.

7. Verfahren nach einem der Ansprüche 1 bis 6, wobei das System der Mehrsäulenchromatographie eine Vielzahl von Säulen und fluidische Verbindungen zwischen den Säulen umfasst, und wobei die Geschwindigkeit der Fluide in den fluidischen Verbindungen zwischen den Säulen grösser als 0,5 m/s, vorzugsweise grösser als 1 m/s und noch bevorzugter grösser als 1,5 m/s ist.

8. Anlage zur Herstellung einer Fruktosezusammensetzung, umfassend:
- eine Zufuhrlinie der anfänglichen Zusammensetzung (21), umfassend Glukose;
- einen ersten Verdampfer (202), der von der Zufuhrlinie der anfänglichen Zusammensetzung (21) versorgt wird,
- eine Sammellinie der konzentrierten anfänglichen Zusammensetzung (22) am Ausgang des ersten Verdampfers (202);
- einen Reaktor zur Isomerisation (203) der Glukose zu Fruktose, versorgt durch die Sammellinie der konzentrierten anfänglichen Zusammensetzung (22);
- eine Sammellinie der Zwischenzusammensetzung (23) am Ausgang des Reaktors zur Isomerisierung (203);
- ein System der Mehrsäulenchromatographie (205), versorgt durch die Sammellinie der Zwischenzusammensetzung (23);
- eine Sammellinie des Raffinats (27) und eine Sammellinie des Extrakts (29) am Ausgang des Systems der Mehrsäulenchromatographie (205);
- einen zweiten Verdampfer (206), versorgt von einer Versorgungslinie (29a), die aus der Sammellinie des Extrakts (29) stammt;
- eine Sammellinie der Fruktosezusammensetzung (26) am Ausgang des zweiten Verdampfers (206);
in der die Sammellinie der Zwischenzusammensetzung (23) einen Verdampfer nicht versorgt; und
in der die Sammellinie des Raffinats (27) mit der die Zufuhrlinie der anfänglichen Zusammensetzung (21) verbunden ist, um den ersten Verdampfer (202) zu versorgen.

9. Anlage nach Anspruch 8, umfassend eine Einheit zur Entfärbung des Extrakts, die sich zwischen dem System der Mehrsäulenchromatographie (205) und dem zweiten Verdampfer (206) befindet, umfassend vorzugsweise ein lonentauschharzbett und/oder Aktivkohle sowie vorzugsweise eine Einheit zum sterilen Filtrieren.

10. Anlage nach Anspruch 8 oder 9, umfassend eine Einheit zur Demineralisierung zwischen dem Reaktor zur Isomerisierung (203) und dem System der Mehrsäulenchromatographie (205).

11. Anlage nach einem der Ansprüche 8 bis 10, umfassend eine Ableitungslinie der Zwischenzusammensetzung (25), die aus der Sammellinie der Zwischenzusammensetzung (23) stammt und mit der Sammellinie des Extrakts (29) verbunden ist, um den zweiten Verdampfer (206) durch die Versorgungslinie (29a) zu versorgen.

12. Anlage nach einem der Ansprüche 8 bis 11, wobei das System der Mehrsäulenchromatographie (205) 4 bis 6 Zellen umfasst; und/oder Säulen umfasst, die eine Länge von 1,0 bis 2,6 m, vorzugsweise von 1,4 bis 2,0 m aufweisen.

13. Anlage nach einem der Ansprüche 8 bis 12, wobei das System der Mehrsäulenchromatographie (205) eine Vielzahl von Säulen und fluidische Verbindungen zwischen den Säulen umfasst, und wobei das Volumen der fluidischen Verbindungen zwischen den Säulen kleiner als 10 %, vorzugsweise kleiner als 5 % und vorzugsweise kleiner als 3 % des Volumens der Säulen ist.

## Claims

1. A method for producing a fructose composition comprising the following successive steps:
- provision of an initial composition comprising glucose;
- concentration of the initial composition by evaporation of water to obtain a concentrated initial composition;
- isomerization of glucose to fructose from the concentrated initial composition, making it possible to obtain an intermediate composition;
- purification of the intermediate composition in a multicolumn chromatography system, making it possible to obtain a glucose-rich raffinate and a fructose-rich extract;
- concentration of the extract by evaporation of water;
wherein the intermediate composition is not subjected to a concentration step by evaporation of water between the isomerization step and the purification step, and
the raffinate is recycled and added to the initial composition prior to the concentration step of the initial composition.

2. The method of claim 1, further comprising a step of removing residual color from the extract prior to the concentration step of the extract, preferably by ion exchange resin and/or by activated carbon as well as, preferably, a sterile filtration step.

3. The method of claim 1 or 2, further comprising a demineralization step between the isomerization step and the chromatography step.

4. The method of any one of claims 1 to 3, wherein the purification is carried out at a temperature greater than or equal to 50°C, preferably 55 to 65°C, and more preferably about 60°C.

5. The method of any one of claims 1 to 4, wherein the intermediate composition which is subjected to the purification step has a dry matter mass concentration of 45 to 55%, and preferably about 50%.

6. The method of any one of claims 1 to 5, wherein the dry matter mass concentration of the intermediate composition varies from less than 5%, and preferably remains essentially constant, between the end of the isomerization step and the beginning of the purification step.

7. The method of any one of claims 1 to 6, wherein the multicolumn chromatography system comprises a plurality of columns and intercolumn fluidic links, and wherein the velocity of the fluids in the intercolumn fluidic links is greater than 0.5 m/s, preferably greater than 1 m/s, and more preferably greater than 1.5 m/s.

8. An installation for producing a fructose composition comprising:
- a glucose-comprising initial composition feed line (21);
- a first evaporator (202) fed by the initial composition feed line (21);
- a concentrated initial composition collection line (22) at the outlet of the first evaporator (202);
- a glucose to fructose isomerization reactor (203) fed by the concentrated initial composition collection line (22);
- an intermediate composition collection line (23) at the outlet of the isomerization reactor (203);
- a multicolumn chromatography system (205) fed by the intermediate composition collection line (23);
- a raffinate collection line (27) and an extract collection line (29) at the outlet of the multicolumn chromatography system (205);
- a second evaporator (206) fed by a feed line (29a) coming from the extract collection line (29);
- a fructose composition collection line (26) at the outlet of the second evaporator (206);
wherein the intermediate composition collection line (23) does not feed an evaporator; and
wherein the raffinate collection line (27) is connected to the initial composition feed line (21) in order to feed the first evaporator (202).

9. The installation of claim 8, comprising an extract decolorization unit located between the multicolumn chromatography system (205) and the second evaporator (206), preferably comprising a bed of ion exchange resin and/or activated carbon, as well as, preferably, a sterile filtration unit.

10. The installation of claim 8 or 9, comprising a demineralization unit between the isomerization reactor (203) and the multicolumn chromatography system (205).

11. The installation of any one of claims 8 to 10, comprising an intermediate composition bypass line (25) from the intermediate composition collection line (23) and connected to the extract collection line (29), in order to feed the second evaporator (206) via the feed line (29a).

12. The installation of any one of claims 8 to 11, wherein the multicolumn chromatography system (205) comprises from 4 to 6 cells; and/or comprises columns having a length of 1.0 to 2.6 m, preferably 1.4 to 2.0 m.

13. The installation of any one of claims 8 to 12, wherein the multicolumn chromatography system (205) comprises a plurality of columns and intercolumn fluidic links, and wherein the volume of the intercolumn fluidic links is less than 10%, preferably less than 5%, and preferably less than 3% of the volume of the columns.
